# EUROPEAN PATENT SPECIFICATION

(11) **EP 4 178 410 B1**
(45) Date of publication and mention of the grant of the patent: **31.12.2025**
(21) Application number: 21746652.3
(22) Date of filing: 05.07.2021
(51) Int. Cl.: A61B 1/00, A61B 1/005

(54) **DEFLECTION CONTROL MECHANISM FOR A STEERABLE FLEXIBLE ENDOSCOPE, STEERABLE FLEXIBLE ENDOSCOPE, ENDOSCOPE ASSEMBLY SET, AND METHOD FOR ASSEMBLING A FLEXIBLE ENDOSCOPE**
ABLENKSTEUERUNGSMECHANISMUS FÜR EIN LENKBARES FLEXIBLES ENDOSKOP, LENKBARES FLEXIBLES ENDOSKOP, ENDOSKOPANORDNUNGSSATZ UND VERFAHREN ZUR MONTAGE EINES FLEXIBLEN ENDOSKOPS
MÉCANISME DE COMMANDE DE DÉVIATION POUR UN ENDOSCOPE FLEXIBLE ORIENTABLE, ENDOSCOPE FLEXIBLE ORIENTABLE, ENSEMBLE D'ASSEMBLAGE D'ENDOSCOPE ET PROCÉDÉ D'ASSEMBLAGE D'UN ENDOSCOPE FLEXIBLE

(30) Priority: 08.07.2020 DE 102020118047
(43) Date of publication of application: 17.05.2023
(73) Proprietor: Karl Storz SE & Co. KG, 78532 Tuttlingen (DE)
(72) Inventor: MÄESARAPU, Alar, 78532 Tuttlingen Baden-Württemberg (DE); TOMBERG, Johan, 78532 Tuttlingen Baden-Württemberg (DE)
(86) International application number: PCT/EP2021/068513
(87) International publication number: WO 2022/008442

(56) References cited:
- JP-A- 2000 051 147
- JP-A- 2009 172 019
- US-A1- 2014 357 954

## Description

The present invention relates to a deflection control mechanism for a steerable flexible endoscope, to a steerable flexible endoscope having such a deflection control mechanism, to an endoscope assembly set, and to a method for assembling a flexible endoscope.

Flexible endoscopes comprise a flexible elongated shaft that is configured for being inserted into an internal cavity of a human or animal body or any other object. In a distal (i.e. distant from a user) end section of the shaft an imaging optics is arranged for generating an image of a scene in the cavity being observed. The endoscopic image generated may be transmitted to a proximal (i.e. close to the user) end of the shaft by a bundle of optical fibers to be picked up by an electronic image sensor positioned in a handpiece arranged at the proximal end of the shaft, or an electronic image sensor may be arranged in the distal end section of the shaft, the image signal being transmitted by electric lines arranged inside the shaft. A flexible endoscope usually comprises an illumination system for illuminating the cavity to be observed, and one or more instrument and/or fluid channels extending from the handpiece through the shaft to its distal end.

Flexible endoscopes are typically employed for observing a cavity being accessed through a curved path, or to observe the interior of an organ that itself has a curved shape such as the intestine, for example. Due to its flexibility the flexible shaft is able to adapt during insertion to the curvature of the access path or the organ. Frequently, however, it is desirable to be able to actively deflect or bend the distal end section of the flexible shaft out of a straight alignment into a bent or articulated shape, in order to facilitate insertion through a curved access path or organ. Moreover, as a viewing direction of the imaging optics generally is determined by a direction of the distal end section of the endoscope shaft, active deflection of the distal end section permits observing a desired partial area of the cavity, and may permit substantially complete observation of the surface of the cavity by varying a deflection angle of the distal end section. Endoscopes permitting active deflection or bending of a section of the flexible shaft are usually denoted "steerable endoscopes".

A steerable or deflectable section of the shaft of a steerable endoscope typically has a supporting structure of consecutively jointed pivoting elements, for example, wherein the pivoting elements can be tipped toward one another by axially movable control wires. Alternatively, the supporting structure may be formed by one or more elastic bending elements, for example, which can be bent by the action of control wires. The supporting structure is enclosed in a flexible sheath made of a synthetic material, for example. Deflection of the steerable section from an aligned position into a bent or articulated shape in one or an opposite direction can be controlled by reciprocating movement of one pair of counteracting control wires. Frequently, steerable endoscopes permit bending the steerable section in two perpendicular planes by operation of two pairs of control wires. The control wires may be fed along the outside or inside of the pivoting elements and extend through the shaft in a proximal direction into the handpiece where a deflection control mechanism is provided. The deflection control mechanism effects movement of the control wires under user control, for example by turning one or more hand wheels.

Due to the design of the pivoting elements or the elastic bending elements and/or due to requirements of the flexible sheath or other elements of the shaft having a minimal bending radius, articulation of the steerable section is limited to a maximal permissible deflection angle. During operation of the steerable endoscope, however, a user normally has no direct visual control of the articulation of the steerable section. Thus steerable endoscopes have been provided having a deflection control mechanism that comprises a mechanical stop, in order to avoid exceeding the maximal permissible deflection angle and thus to avoid breakage or inadmissible deformation of components.

Flexible steerable endoscopes are available having a large variety of shaft lengths, shaft diameters, internal designs including instrument and/or irrigation channels, and shaft stiffnesses, as well as lengths, diameters, designs, stiffnesses, and maximal deflection angles of the steerable section. In each case, longitudinal movement of the control wires may be limited to a particular range corresponding to a particular maximal deflection angle. On the other hand, for reasons of cost it would be desirable to employ standardized components for a variety of endoscopes.

European patent EP 2 835 096 B1 discloses a bending angle adjustment mechanism for setting a maximum bending angle of an endoscope. The mechanism is for adjusting a moving amount and a moving range of bending operation wires and chains, in a bending operation mechanism for performing a bending operation of a bending portion of the endoscope. The bending angle adjustment mechanism comprises a stopper member that restricts movement of the bending operation wire, and a screw member that performs positional adjustment by moving a position of the stopper member along a moving direction of the operation wire. According to European patent application EP 3 222 195 A1 an endoscope has a bending adjustment unit comprising a stopper portion and a contact part coming into contact with the stopper portion, thereby regulating movement of a chain to which bending wires are coupled. A bending operation mechanism and the bending adjustment unit are provided on a flat surface side of a main frame that is formed in a plate shape.

JP 2000 051 147A discloses the preamble of claim 1.

The prior art deflection control mechanisms have proven to be not optimal regarding tactile feedback to a user, such that the user clearly feels when a maximal permissible deflection angle has been reached. In particular, even if the stop has been reached, limiting the longitudinal movement of the control wires, the user may have the impression that increasing the force applied to a hand wheel of the deflection control mechanism may further increase the bending angle, thus increasing the risk of failure of the endoscope. Moreover, it is desirable to facilitate assembly and maintenance of the deflection control mechanism.

It is therefore an object of the present invention to provide a deflection control mechanism for a steerable flexible endoscope in which one or more of the above mentioned drawbacks are avoided or alleviated. In particular, it is an object of the invention to provide a deflection control mechanism having a more clearly defined and more rigid stop, giving improved tactile feedback to a user when a maximal deflection angle has been reached, and/or to provide a deflection control mechanism being improved regarding assembly and/or maintenance. Further objects of the invention are to provide a steerable flexible endoscope having an improved deflection control mechanism, a corresponding endoscope assembly set, and an improved method for assembling a flexible endoscope.

These objects are met by a deflection control mechanism according to claim 1, by a steerable flexible endoscope according to claim 13, by an endoscope assembly set according to claim 14, and by a method according to claim 15. Particular embodiments of the present invention are indicated in the dependent claims.

The present invention relates to medical endoscopes, i.e. to endoscopes designed for medical applications, such as, for example, minimally invasive surgery and/or medical examinations. A user of the endoscope therefore typically is a surgeon conducting an endoscopic intervention. However, the present invention also relates to endoscopes designed for non-medical purposes, for example to industrial endoscopes or borescopes.

According to an aspect of the present invention, a deflection control mechanism is configured for controlling a deflection or articulation or a bending angle of a steerable section of a shaft of a steerable flexible endoscope. The shaft of the endoscope is an elongate, flexible shaft that is configured for being inserted into an internal cavity of a human or animal body or another object, for example a technical object, and comprises a steerable section that can be actively bent or deflected with respect to other sections of the shaft. In particular, the steerable section may be actively deflectable with respect to an adjacent section of the flexible shaft under user control. The steerable section may form a distal end section of the shaft, or a section close to the distal end of the shaft, for example. The steerable section may comprise an imaging optics, or an imaging optics may be arranged in an end cap arranged at a distal end of the steerable section, such that by deflecting the steerable section a viewing direction of the imaging optics can be varied. The endoscope may further comprise a handpiece connected to a proximal end section of the shaft, the handpiece having control elements for controlling various functions of the endoscope. An endoscopic image generated by the imaging optics may be transmitted by optical or electronic means to the handpiece. The deflection control mechanism is connectable to the proximal end section of the shaft, and may be accommodated in the handpiece.

The steerable section of the shaft of the flexible endoscope is deflectable by longitudinal movement of at least one pair of counteracting control wires. The steerable section may have a supporting structure of pivoting or elastic elements, for example, which can be angled or bent due to a longitudinal force or a bending moment exerted by the control wires. Typically, the two wires of the pair of counteracting control wires are arranged on opposing sides of the supporting structure, preferably approximately symmetrically to a longitudinal axis of a respective part of the shaft. For controlling the deflection or bending of the steerable section the control wires are movable relative to the shaft substantially in their respective longitudinal direction, which direction usually is parallel to the axial direction of the respective part of the shaft. Thus, when a first one of the two wires of the pair is pulled in the proximal direction for deflection to one side, the other one will move in the distal direction; for deflection to the opposite side, the other one of the two wires is pulled in the proximal direction, while the first one will move in the distal direction. Deflection of the steerable section from an aligned position into a bent or articulated shape in one or an opposite direction, or vice versa, can be controlled by such opposing or counteracting movement of the two wires of the pair of control wires. The control wires are guided in the shaft to the proximal end of the shaft, being operable in such opposing or counteracting manner by the deflection control mechanism connectable to the proximal end of the shaft. The control wires may also be denoted pull wires, referring to a pulling action of the wires, i.e. a force directed in a proximal direction, or push-pull wires, if the wires are configured similar to Bowden wires for also exerting a force in a distal direction.

The deflection control mechanism comprises a support structure, a drive wheel rotatably supported in or on the support structure, and an elongate traction element. The drive wheel is rotatably supported in or on the support structure, for example with one or more bearings, having a rotatable axle or shaft or an axle fixed to the support structure. The support structure preferably is rigid. The support structure may comprise, for example, a non-rotating axle of the drive wheel, a cage structure in which the drive wheel is housed, and/or elements of a housing of the handpiece. The traction element comprises a flexible section and a first and a second coupling section. The flexible section may be configured as a chain, for example. The flexible section has a first end and a second end opposite the first end. The first coupling section is connected to the first end, and the second coupling section is connected to the second end of the flexible section. Preferably the flexible section and the first and second coupling sections have high stiffness in a longitudinal direction.

The flexible section of the traction element engages with the drive wheel, in particular with a circumferential surface of the drive wheel, such that by turning the drive wheel the first and second coupling sections are moved lengthwise. The drive wheel may be configured as a sprocket and the flexible section as a chain, the teeth of the sprocket engaging with the chain segments to move the chain in its length direction. Preferably the flexible section is wound around the drive wheel, encircling an angle of about 180° or an angle of about 180° plus an integer multiple of 360°, such that the traction element approximately is arranged in a "U" shape, or an angle exceeding 180°, for example an angle between 180° and 200° or 220°, to improve coupling with the drive wheel while providing a compact design. Both coupling sections may be at least approximately parallel to each other, defining a common longitudinal direction. By rotating the drive wheel the coupling sections therefore are moved in the longitudinal direction in an opposing manner. The drive wheel may be coupled with a hand wheel, for example, for being rotated manually by the user. Alternatively or additionally, the drive wheel may be coupled or configured for being coupled with a motor for being rotated in a motorized or robotic manner. Thus, for example, the flexible endoscope may be configured for being arranged at a robotic arm, such that the deflection control mechanism can be operated for controlling articulation of the steerable section in a robotic manner.

The first and second coupling sections are configured for being coupled with the at least one pair of counteracting control wires. To this end the first and second coupling sections each comprise a fixation element for fixing a respective proximal end of each wire of the at least one pair of counteracting control wires. The fixation element may be configured as a rectangular fixation block, for example, comprising a longitudinal bore in which the proximal end section of the respective control wire can be clamped or in which a clamping element or a ferrule holding the wire can be fixed. When connecting the deflection control mechanism to the shaft of the steerable endoscope, both wires are fixed in the respective fixation element. Thus, by rotating the drive wheel the deflection angle of the steerable section of the shaft is controllable.

Further, at least one of the first and second coupling sections comprises a movable stop element co-operating with a fixed stop of the support structure to limit a range of movement of the respective coupling section and thus to limit a range of longitudinal movement of the respective control wire. As the movable stop element is comprised by the respective coupling section, during operation of the deflection control mechanism it is co-moving with the respective fixation element and with the respective control wire fixed to the fixation element. The fixed stop or stopper is formed or supported by the support structure and therefore remains in a fixed position during operation. The movable stop element co-operates with the fixed stop to define a limit to a movement of the traction element and thus to limit the movement of the control wires. In particular, the movable stop element may be stopped by contacting the fixed stop when the end of a permissible range of movement of the control wires has been reached. In this way deflection of the steerable section is limited to a maximal deflection or articulation angle, at least to one side of the shaft. Preferably, the first and the second coupling sections each comprise a movable stop element co-operating with a respective fixed stop of the support structure to limit a range of movement of the respective coupling section, thus limiting a range of movement of the control wires in proximal as well as in distal direction. In this way deflection of the steerable section can be limited to a maximal deflection angle to one side as well as to an opposite side of the shaft.

According to this aspect of the invention, the movable stop element of the at least one of the first and second coupling sections is adjustably connected to the fixation element of the respective coupling section. Thus a longitudinal position of the movable stop element relative to the fixation element can be adjusted. In particular, the deflection control mechanism is configured such that the relative position of the movable stop element can be adjusted during assembly or maintenance. On the other hand, the fixed stop generally has a position predetermined by design and thus is not adjustable. Due to the co-operation of the movable stop element with the fixed stop, a limit of movement of the respective control wire can be adjusted in this way, at least in either proximal or distal direction. Most preferably, the first and second coupling sections each comprise a movable stop element co-operating with a respective fixed stop of the support structure, and both movable stop elements are adjustably connected to the respective fixation element of the respective coupling section. That is the first coupling section has a first movable stop element co-operating with a first fixed stop, and the second coupling section has a second movable stop element co-operating with a second fixed stop, wherein the first movable stop is adjustably connected to the fixation element of the first coupling section, and the second movable stop is adjustably connected to the fixation element of the second coupling section. In this way the maximal range of movement of the control wires in proximal as well as in distal direction can be adjusted, preferably during assembly or maintenance.

The deflection control mechanism may comprise further elements, such as a brake, for example. A brake permits blocking one or both control wires of the at least one pair of counteracting control wires in their respective longitudinal positions, such that the steerable section of the endoscope is fixedly held at a corresponding deflection angle. The deflection control mechanism may be configured for effecting movement of more than one pair of counteracting control wires. Preferably the deflection control mechanism is operable manually by turning one or more hand wheels, and/or may be operable in motorized or robotic manner. A shaft coupling may be provided at a distal side of the handpiece or the deflection control mechanism for coupling the endoscope shaft to the handpiece and/or the deflection control mechanism.

Depending on a particular intended application, steerable endoscopes have different shaft diameters, different shaft lengths, different maximal deflection angles, and/or may differ in further properties, and therefore have different permissible ranges of longitudinal movement of the control wires. Due to the movable stop element being adjustably connected to the respective fixation element of one or both coupling sections, a range of movement of the control wires can be limited such that a permissible maximal deflection angle of a particular endoscope shaft cannot be exceeded. Thus the deflection control mechanism may be adaptable to a variety of shafts of steerable flexible endoscopes and/or permit adjustments due to manufacturing tolerances and service requirements. Moreover, due to the movable stop element being adjustable, a more compact design can be achieved providing increased space for other components in the handpiece, and possesses increased stiffness and rigidity. A user operating the steerable endoscope may thus receive a clearer tactile feedback when a limit of the permissible range of deflection angles has been reached. In particular, when reaching the limit, a hand wheel being turned manually by the user for operating the deflection control mechanism cannot be turned perceivably further even by exerting an increased torque on the hand wheel. Thereby operation of the deflection control mechanism can be facilitated. Further, due to increased stiffness, breakage of components during operation can be more safely avoided.

Preferably the movable stop element of the at least one of the first and second coupling sections is configured as a rectangular block. In particular, the movable stop element may have an at least substantially rectangular cross section, thereby providing a simple design and permitting guiding the movable stop element for longitudinal movement in rotation-free manner in a corresponding guiding channel. The movable stop element may have a flat stop face for co-operating with the fixed stop of the support structure by contacting the fixed stop when the end of a range of travel has been reached. The flat stop face may be formed perpendicular to the longitudinal direction of the coupling section, and/or the movable stop element may be roughly rectangular in a longitudinal section. In this way the mechanical stop indicating the limit of a permissible deflection can be formed in a particularly well-defined and rigid manner. Preferably the stop face is arranged at a proximal side of the movable stop element.

According to the invention the movable stop element of the at least one of the first and second coupling sections is mounted on a threaded rod connected to the fixation element of the respective coupling section. The threaded rod extends substantially in the longitudinal direction of the respective coupling section, the movable stop element being adjustable in the longitudinal direction. Thus a longitudinal position of the movable stop element relative to the fixation element, and thus relative to the proximal end of the respective control wire, can be adjusted. In particular, the movable stop element can be adjusted by turning or screwing it on the threaded rod in proximal or distal direction. During operation of the deflection control mechanism the movable stop element is co-moving with the fixation element. In case that the movable stop element is configured as a substantially rectangular block, its position on the threaded rod can be fixed by guiding the block in a correspondingly shaped channel, thus providing a particularly simple design.

According to a particularly advantageous embodiment the at least one of the first and second coupling sections has a tensioning mechanism for pre-tensioning the at least one pair of control wires, such as to avoid buckling or play of the control wires and thus improving controllability of the articulation. In particular, the flexible section of the traction element may be connected to a proximal end of the threaded rod, for example by means of a hinge, and the threaded rod is connected at its distal end to the fixation element, forming the tensioning mechanism, tensioning being accomplished by screwing the threaded rod into a corresponding threaded bore of the fixation element. Moreover, in a similar manner as the movable stop element, the fixation element may be configured as a substantially rectangular block, being guided in a correspondingly shaped channel, to keep a chosen tensioning state. In this way a particularly simple and stable design can be achieved. Alternatively the other one of the first and second coupling sections may be provided with the tensioning mechanism. Preferably each one of the first and second coupling sections comprises a movable stop element adjustably mounted on a respective threaded rod and a tensioning mechanism that are configured as described.

According to a further aspect of the present invention, the support structure comprises at least one base plate arranged substantially in a base plane extending at least approximately perpendicular to a rotational axis of the drive wheel. The base plate may be substantially plane, having protrusions and/or bores, and may have a larger extension in the longitudinal direction, i.e. parallel to the direction of movement of the coupling sections, than in a transverse direction. In this way a particularly simple and stable arrangement can be provided.

In accordance with an advantageous embodiment, the flexible section and the first and second coupling sections of the traction element extend at least approximately in or adjacent and substantially parallel to the base plane. In particular, the longitudinal movement of the coupling sections may be effected parallel to the base plane. Further in accordance with this embodiment, the base plate is arranged substantially between the first and second coupling sections of the traction element, and the base plate supports the fixed stop of the support structure. In particular, the traction element may be arranged substantially in a "U" shape, wherein the fixed stop is situated on the base plate at an inner side of the "U"; therefore the fixed stop can be arranged close to the drive wheel and can be more directly and rigidly connected to an axle of the drive wheel. The fixed stop may be an element connected to the base plate, or the fixed stop may be formed by a protrusion or shoulder of the base plate and thus be integral with the base plate. In this way a particularly stiff and rigid design can be achieved, providing a still more rigid and clearer definition of the mechanical stop provided by the movable stop element co-operating with the fixed stop. Moreover, the base plate may be configured such that the fixed stop approximately defines a permissible range of movement of the control wires, while the movable stop element is adjustable for fine-tuning and/or service re-adjustment of the range of movement.

Advantageously the support structure comprises a cage structure in which the drive wheel is rotatably supported, for example on a fixed axle, the axle being held in the cage structure. Preferably the cage structure is rigid, the axle being rigidly fixed in the cage structure. Further the support structure may comprise a housing of the deflection control mechanism, the housing being rigidly, directly or indirectly, connected to the base plate and being rigidly connected to the cage structure or at least supported on the cage structure in the longitudinal direction. In this way a particularly stable and rigid connection between a bearing of the drive wheel and the fixed stop can be accomplished, further improving rigidity of the mechanical stop.

Preferably the base plate is formed of metal, for example as one piece of sheet metal, and the support structure comprises at least one plastic plate arranged substantially parallel and adjacent to the base plate. The plastic plate may be substantially plane and may have protrusions and/or bores for alignment and fixation with respect to the base plate. The plastic plate may have a larger extension in the longitudinal direction than in a transverse direction. The support structure may comprise two plastic plates arranged on either side of the base plate. Preferably the one or two plastic plates is or are arranged between the first and second coupling sections, i.e. when the traction element forms a "U" shape, in an inner space of the "U". Advantageously the one or two plastic plates is or are arranged immediately adjacent to or abut the base plate. In particular, one plastic plate may be stacked upon the base plate and, in case that two plastic plates are provided, the base plate stacked on the other plastic plate, providing a friction fit. In this way stability of the support structure can be improved, and a cheap and simple design can be provided.

Further preferably, the one or two plastic plates may be rigidly fixed to the base plate by one or more pins, bolts and/or screws, for example, to increase rigidity. Moreover, a housing of the deflection control mechanism may be rigidly fixed to the arrangement of stacked plates by the one or more pins, bolts and/or screws. In this way a particularly stable and stiff design can be achieved, wherein the base plate and the one or two plastic plates can be held precisely together to form a sub-assembly that is manufactured beforehand, and/or the sub-assembly can be connected precisely and rigidly to a body of the handpiece.

In advantageous manner the at least one plastic plate may form a guideway for the longitudinal movement of the movable stop element of the at least one of the first and second coupling sections. In particular, the movable stop element may be guided in a channel an inner wall of which is formed by the at least one plastic plate. Likewise the fixation block may be guided by the plastic plate or in the same channel. In this way friction can be reduced that could arise in the movement of the coupling section during operation of the deflection control mechanism.

In accordance with a preferred embodiment of the invention, the deflection control mechanism comprises a further drive wheel rotatably supported in or on the support structure, preferably co-axially with the aforementioned drive wheel, and a further elongate traction element having a flexible section, a first coupling section connected to a first end of the flexible section, and a second coupling section connected to a second end of the flexible section. The flexible section of the further traction element engages with the further drive wheel, and the first and second coupling sections of the further traction element each comprise a fixation element for fixing a respective proximal end of each wire of at least one further pair of control wires, wherein at least one of the first and second coupling sections of the further traction element comprises a movable stop element co-operating with a further fixed stop of the support structure and wherein this movable stop element is adjustably connected to the fixation element of the respective coupling section of the further traction element. The further drive wheel and the further traction element may be configured like the drive wheel and the traction element described above. In particular, the deflection control mechanism according to the present embodiment may be considered to comprise, in addition to the elements of the deflection control mechanism according to the previously described embodiments, further elements constituting a further deflection control mechanism, having a common support structure. The deflection control mechanism may be configured for controlling deflection of the steerable section of an endoscope shaft that comprises two pairs of control wires for controlling deflection of the steerable section in two different planes, a first pair of counteracting control wires being operated by rotating the drive wheel and moving the traction element as described above for controlling deflection in a first plane, and the further pair of counteracting control wires being operated by rotating the further drive wheel and moving the further traction element for controlling deflection in a second plane. The first and second planes may be at an angle of 90° to each other. Thus, the steerable section can be deflected in any arbitrary direction.

As described above, the support structure may comprise a base plate arranged approximately in a base plane extending substantially perpendicular to a rotational axis of the drive wheel, wherein the flexible section and the first and second coupling sections of the traction element extend substantially in or adjacent to the base plane, the base plate being arranged substantially between the first and second coupling sections and supporting the fixed stop of the support structure. Thus, the base plate and the traction element may be considered to form a first layer. According to an advantageous embodiment, the support structure comprises a further base plate arranged approximately in a further base plane extending substantially perpendicular to a rotational axis of the further drive wheel, wherein the flexible section and the first and second coupling sections of the further traction element extend substantially in or adjacent to the further base plane, the further base plate being arranged substantially between the first and second coupling sections of the further traction element and supporting the further fixed stop of the support structure. Advantageously, the further base plate may be arranged substantially parallel to and rigidly connected to the aforementioned base plate. The further base plate and the further traction element may thus be considered to form a second layer of the deflection control mechanism. Preferably, the base plate and the further base plate are connected to each other by one or more bolts and/or screws, and may be stacked upon one another, including further plates that may be configured as described above, for example plastic plates. The first and the second layer may therefore be considered to be stacked upon one another, providing increased rigidity of the overall arrangement of the deflection control mechanism, the deflection control mechanism being suitable for controlling two pairs of control wires.

According to a further aspect of the present invention, a steerable flexible endoscope has an elongate flexible shaft comprising a steerable section that is deflectable or bendable by longitudinal movement of at least one pair of counteracting control wires, wherein the flexible endoscope further comprises a deflection control mechanism configured as described above. In particular, the endoscope may comprise a handpiece connected to a proximal end of the flexible shaft, the handpiece accommodating the deflection control mechanism, wherein the proximal ends of the wires of the at least one pair of counteracting control wires are fixed in the respective fixation elements of the deflection control mechanism. Most preferably, the steerable section is deflectable in two perpendicular planes by movement of two pairs of control wires, and the deflection control mechanism is configured for operating two pairs of control wires for controlling deflection in the two perpendicular planes as described before. In accordance with a still further aspect of the present invention, an endoscope assembly set is provided that comprises at least a first and a second elongate flexible shafts, each elongate flexible shaft comprising a steerable section that is deflectable by movement of at least one pair of counteracting control wires, the first and second elongate shafts having different permissible ranges of longitudinal movement of the control wires. The first and second elongate shafts may have different shaft diameters, different shaft lengths, different maximal deflection angles, and/or may differ in further properties, resulting in different permissible ranges of longitudinal movement of the control wires. The endoscope assembly set further comprises a deflection control mechanism configured as described above, wherein the deflection control mechanism is connectable to the proximal end of either one the first and second flexible shafts and is adaptable for controlling deflection of the steerable section of the respective flexible shaft, and wherein a longitudinal position of the movable stop element of at least one of the first and second coupling sections of the traction element of the deflection control mechanism is adjustable such that the deflection control mechanism is adaptable to the respective permissible range of longitudinal movement of the control wires of the respective flexible shaft. Thus the deflection control mechanism can be combined with either of the flexible shafts, being adjusted during assembly. Preferably, the assembly set comprises three or more flexible shafts, the deflection control mechanism being adaptable to any one of the flexible shafts by adjusting the at least one movable stop element. Alternatively or additionally, the endoscope assembly set may include a multiplicity of base plates, each having a fixed stop at a position roughly corresponding to a permissible range of movement of the control wires of a respective one of the flexible shafts, each base plate being configured to be included in a deflection control mechanism as described above, while fine adaptation of the range of movement can be accomplished by adjusting the movable stop element. In this way an assembly set can be provided having improved versatility, and reduced cost.

The present invention further relates to a method for assembling a flexible endoscope, the flexible endoscope having an elongate flexible shaft comprising a steerable section that is deflectable by movement of at least one pair of counteracting control wires. In accordance with the method, the elongate flexible shaft of the endoscope is provided, and a deflection control mechanism is provided, the deflection control mechanism being configured as described above, which may include an assembled stack of plates as described above. The assembled stack of plates may comprise a base plate having a fixed stop being approximately adapted to a permissible range of movement of the control wires of the flexible shaft. Further, a longitudinal position of the movable stop element of at least one of the first and second coupling sections of the traction element of the deflection control mechanism is adjusted, preferably finely adjusted, to the permissible range of longitudinal movement of the control wires of the flexible shaft, such that the deflection control mechanism is adapted for controlling deflection of the steerable section of the elongate flexible shaft while limiting the movement of the control wires to the permissible range. Thereafter, or before the aforementioned step, the deflection control mechanism is connected to a proximal end of the elongate flexible shaft. The proximal ends of both wires of the at least one pair of counteracting push-pull wires may be fixed in the respective fixation elements of the first and second coupling sections of the deflection control mechanism before or after adjusting the movable stop element. After adjusting the movable stop element, a housing of the deflection control mechanism may be mounted. Thus the flexible endoscope is easy to assemble, employing cheap and simple components.

The deflection control mechanism may comprise further features, such as a brake mechanism as disclosed in the co-pending patent application "Deflection control mechanism for a steerable flexible endoscope, steerable flexible endoscope, and method for controlling a flexible endoscope" (internal file number P01722), and/or a plate structure as disclosed in the co-pending patent application "..." (internal file number KST100), both filed by the same applicant on the same day as the present application.

The features of the invention as mentioned above and as described below apply not only in the combinations mentioned but also in other combinations or alone, without leaving the scope of the present invention.

Further aspects of the present invention will be apparent from the figures and from the description of a particular embodiment that follows.
Fig. 1 shows a steerable flexible endoscope in an overall view;
Fig. 2 shows a deflection control mechanism in accordance with an exemplary embodiment of the present invention;
Fig. 3 shows the deflection control mechanism of Fig. 2 with the housing removed;
Fig. 4 shows the deflection control mechanism as in Fig. 3, but in an enlarged, horizontal longitudinal sectional view;
Fig. 5 shows a proximal part of the deflection control mechanism of Fig. 2 in an enlarged, vertical longitudinal sectional view;
Fig. 6 shows the deflection control mechanism of Fig. 2 with the housing removed, in an enlarged, transverse sectional view;
Fig. 7 shows the arrangement of stacked plates of the deflection control mechanism of Fig. 2.

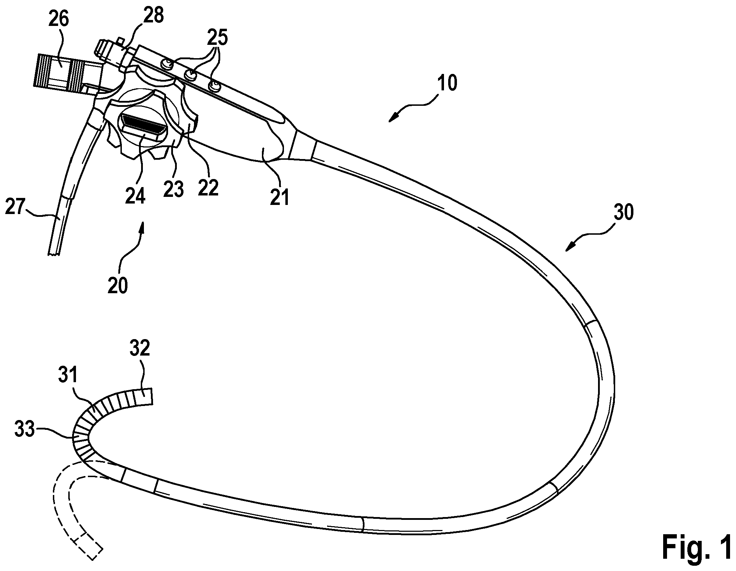

As shown schematically in Fig. 1, a flexible endoscope 10 typically comprises a handpiece 20 and an elongate flexible shaft 30, the handpiece 20 being attached to a proximal end of the shaft 30. The handpiece 20 has an outer housing 21 made of a plastic and/or metallic material. On a lower side of the housing 21 a first hand wheel 22 and a second hand wheel 23 are arranged for controlling a deflection of a steerable section 31 of the shaft 30, as is described below. Typically the first and the second hand wheel 22, 23 are arranged co-axially, and at an exterior side of the second hand wheel 23 a knob 24 for controlling a deflection brake relating to the second hand wheel 23 is provided. Further the handpiece 20 may exhibit a multiplicity of control buttons 25 for controlling various functions of the flexible endoscope 10, such as for controlling the imaging and/or illumination system and/or irrigation and suction pumps, for example. The handpiece 20 may be connectable to an external video unit or a video monitor via connector 26 and to an external light source via light cable 27. Moreover, an instrument port 28 may be provided for inserting endoscopic instruments to be advanced through one or more respective channels to a distal end of the shaft 30 for manipulating tissue or other objects within a cavity into which the shaft 30 can be inserted.

At its distal end the flexible shaft 30 comprises a steerable section 31. The steerable section 31 may form a distal end section of the shaft 30 or, as shown in Fig. 1, may carry a distal end cap 32 which may accommodate an imaging optics and an electronic image sensor for providing an endoscopic image of a cavity into which the shaft 30 is inserted. The image signal generated by the image sensor may be transmitted via electric cables extending through the shaft 30 and the handpiece 20 to the connector 26 for being processed and displayed by an external video unit. The shaft 30 in total is flexible to an extent to be advanced through an endoscopic access or a hollow organ towards a cavity to be observed, being capable of adapting to a curved shape of the access or the organ. The steerable section 31, on the other hand, is capable of being flexed actively by turning the hand wheels 22, 23. To this end the steerable section 31 comprises an inner structure of a multiplicity of consecutive pivoting elements 33, thus being deflectable in one or more planes. The pivoting elements 33 are covered by a flexible tube to form a smooth outer surface, the shaft 30 in total having a uniform cross-sectional shape and diameter.

For controlling the deflection of the steerable section 31 two counteracting control wires (not shown in Fig. 1) are provided extending on opposite sides within the steerable section 31, by a longitudinal movement of which the steerable section 31 can be bent to one or the other side, as indicated symbolically in Fig. 1. The control wires extend along the shaft 30 and are connected at their proximal ends to a deflection control mechanism arranged within the handpiece 20 which can be operated by a user by turning the hand wheels 22, 23 for deflecting the steerable section 31. In the exemplary embodiment shown, the steerable section can be bent within a first plane that corresponds to the plane of the drawing, a deflection angle being controllable by rotating the first hand wheel 22. Further, the steerable section 31 can be bent in a second plane perpendicular to the first plane and perpendicular to the plane of the drawing, a corresponding deflection angle being controllable by rotating the second hand wheel 23.

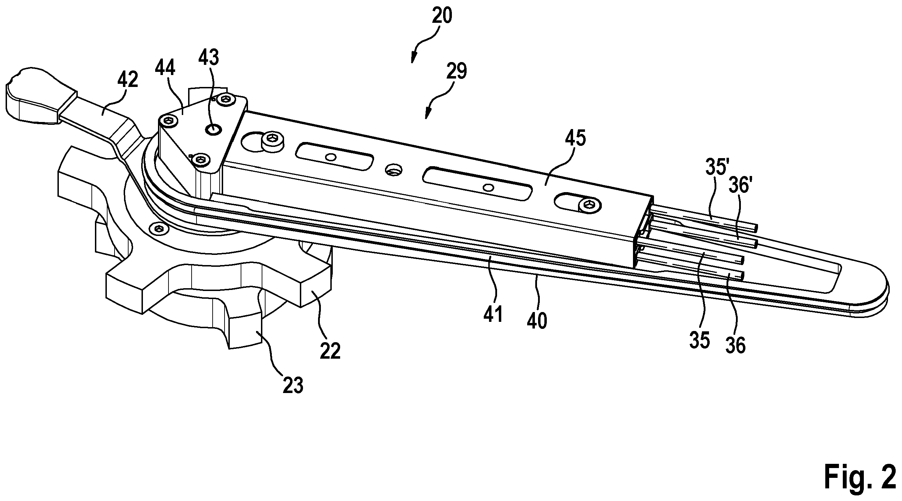

Fig. 2 shows a handpiece 20 of a flexible endoscope in accordance with an exemplary embodiment of the invention, wherein an upper part of the housing 21 of the handpiece 20 has been removed to show the deflection control mechanism 29. The handpiece 20 may comprise further elements not shown in Fig. 2 (see Fig. 1). As depicted in Fig. 2, a lower part of the housing is formed by a handpiece body 40 preferably formed of metal such as stainless steel, for example. At its lateral sides the body 40 carries a seal ring 41 of an elastic seal material to form a sealed connection with the upper part of the housing (not shown in Fig. 2); thus, an interior space of the handpiece 20 accommodating the deflection control mechanism 29 is be sealingly enclosed. At a lower side of the body 40 the first hand wheel 22 and the second hand wheel 23 are rotatably mounted, having a common axis of rotation. The common axis is defined by an axle 43 that is non-rotating and fixedly held in a rigid metal cage 44. The axle defines a rotation axis of a drive wheel (sprocket 59) that is rotationally coupled with the second hand wheel 23, and of a further drive wheel (sprocket 69) rotationally coupled with the first hand wheel 22 (see below). A lever 42 is provided for controlling a deflection brake relating to the first hand wheel 22 (not shown in Fig. 1). Further elements of the deflection control mechanism are covered by a half-shell housing 45 preferably made of metal such as stainless steel. Moreover, in Fig. 2 the proximal end sections of the sheaths 35, 35', 36, 36' of two pairs of counteracting control wires are shown; the control wires (not shown in Fig. 2) continue to the right hand side of the drawing and extend through the shaft 30 of the endoscope to the steerable section 31 (see Fig. 1).

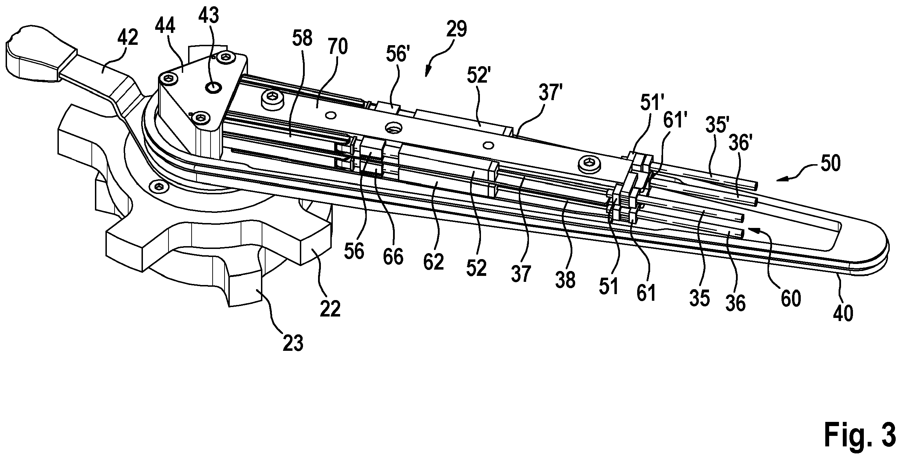

Fig. 3 shows the handpiece 20 as in Fig. 2, but with the half-shell housing 45 removed, showing further details of the deflection control mechanism 29. In Fig. 3 the proximal end sections of the control wires 37, 38 protruding from the respective sheaths 35, 36 are depicted. In corresponding manner the proximal end sections of respective counteracting control wires of the two pairs of control wires protrude from the respective sheaths 35', 36' (only control wire 37' being visible in Fig. 3). In the present example the deflection control mechanism 29 comprises two layers 50, 60, an upper layer 50 being configured for controlling the deflection of the steerable section 31 in a first plane by reciprocating longitudinal movement of the first pair of control wires 37, 37', and a lower layer 60 being configured for controlling a deflection of the steerable section 31 in a second plane, perpendicular to the first plane, by reciprocating movement of the second pair of control wires (of which only control wire 38 is shown in the figures). Both layers 50, 60 are stacked upon one another, being mounted on an upper side of the body 40. The upper layer 50 of the deflection control mechanism 29 is operated by turning the second hand wheel 23, while the lower layer 60 of the deflection control mechanism 29 is operated by turning the first hand wheel 22.

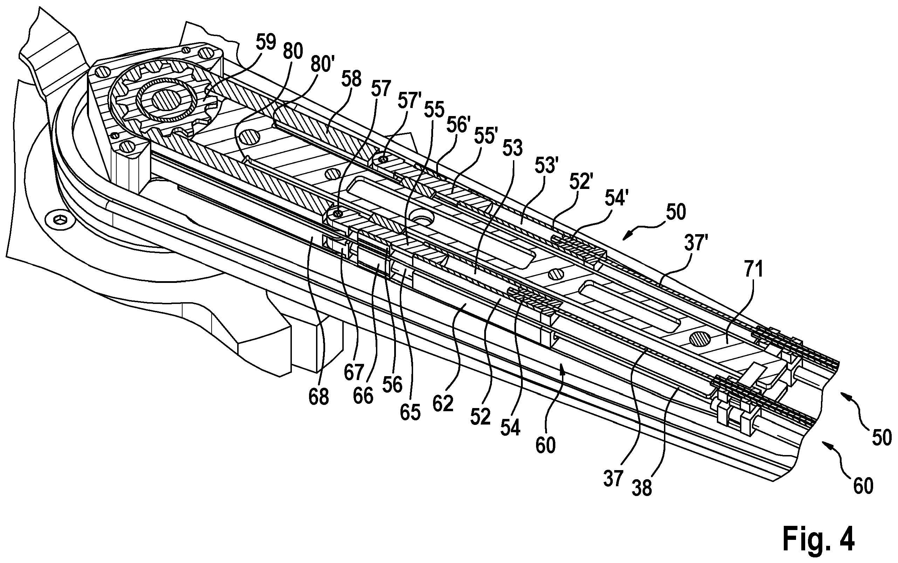

Fig. 4 shows a partial longitudinal sectional view with the cross section being taken approximately in a center plane of the upper layer 50. As can be seen in Figs. 3 and 4 the wire sheaths 35, 35', 36, 36' are each firmly held in a respective wire coil mounting 51, 51', 61, 61'. The control wires 37, 37' operable by the upper layer 50 are each fixed in a respective fixation element (rectangular fixation blocks 52, 52'). The fixation blocks 52, 52' are screwed on a respective threaded rod 55, 55' forming a tensioning mechanism such that the control wires 37, 37' are subjected to a pre-tension. For simplicity, in the following only the upper layer 50 of the deflection control mechanism will be described in detail; the lower layer 60 comprises corresponding elements acting upon the second pair of control wires in a corresponding manner.

As depicted in Fig. 4, the fixation block 52 has a longitudinal bore 53. From a distal side a ferrule 54 is inserted into the bore 53, the ferrule 54 firmly holding the proximal end of the control wire 37. At a proximal side of the block 52 a threaded rod 55 is screwed into the proximal end of the bore 53. For simplicity, the thread formed on the substantially cylindrical surface of the rod 55 is not shown in the figures. By screwing the rod 55 into the bore 53 of the block 52, and correspondingly screwing rod 55' into bore 53' of block 52', control wire 37' being fixed in ferrule 54', the first pair of counteracting control wires 37, 37' can be subjected to a pre-tension, thus avoiding buckling or play of the control wires 37, 37' when the reflection control mechanism is operated. During operation of the deflection control mechanism, the rectangular blocks 52, 52' are guided in respective channels and thus inhibited from rotation, such that de-adjustment of the tensioning mechanism is avoided.

Further, the threaded rod 55 carries a stopping block 56 forming a movable stop element, as is described below. The stopping block 56 has substantially rectangular cross section and is screwed upon the threaded rod 55, the threaded rod 55 extending through a bore of the stopping block 56. During assembly of the endoscope, a longitudinal position of the stopping block 56 with respect to the fixation block 52 can be adjusted by turning the stopping block 56, thus advancing it in proximal or distal direction on the threaded rod 55. During operation of the deflection control mechanism 29, the stopping block 56 is guided in the same channel as the fixation block 52, such that rotation of the stopping block 56 is inhibited, and the longitudinal position of the stopping block 56 relative to the fixation block 52 is fixed.

At its proximal end, the threaded rod 55 is connected by a hinge 57 to a chain 58. The hinge 57 may be considered an end link of the chain 58, having a pin pressed into the hinge 57 and the threaded rod 55. The chain 58 engages with the teeth of the sprocket 59 and is wound around the sprocket 59, enclosing an angle of 180°, thus connecting the rod 55 to a corresponding rod 55' on the other side of the deflection control mechanism. The chain 58 consists of a multiplicity of segments connected to each other by hinges, which are not shown in the figures for simplicity. The hinge 57 has an axis which is vertical in the depiction of Figs. 3 and 4 and thus substantially parallel to the axis of rotation of the sprocket 59. Thus, the chain 58 can be considered a flexible section of a traction element, engaging the drive wheel embodied as sprocket 59. By rotation of the sprocket 59, both ends of the chain 58 and connected coupling sections formed by elements 52-57 and corresponding elements 52'-57', respectively, are moved in opposing directions, thus effecting corresponding opposing movement of the control wires 37, 37'. In this way deflection of the steerable section 31 of the endoscope 10 in a first plane can be controlled (see Fig. 1). The lower layer 60 is configured in a corresponding manner (only block 62, rod 65, block 66, hinge 67, and chain 68 being visible in Figs. 3 and 4), such that deflection of the steerable section 31 of the endoscope 10 in a direction perpendicular to the first plane can be controlled by movement of the second pair of control wires (only control wire 38 being visible in Figs. 3 and 4).

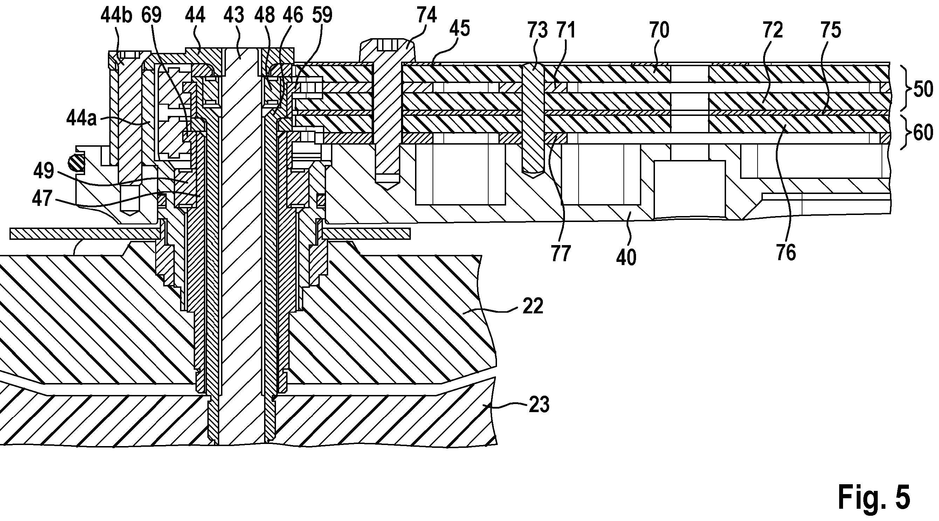

Fig. 5 depicts the proximal end section of the deflection control mechanism 29 in a vertical longitudinal cross section. As can be seen in Fig. 5, the sprocket 59 of the upper layer 50 is rigidly held on an articulation shaft 46 extending to a lower side of the handpiece 20 to the second hand wheel 23 with which it is connected for being rotated by turning the hand wheel 23. Likewise a sprocket 69 of the lower layer 60 of the deflection control mechanism is fixed on a further articulation shaft 47 extending coaxially with the articulation shaft 46 into the first hand wheel 22 with which it is connected for being rotated when the hand wheel 22 is rotated. The articulation shaft 46 and thus the sprocket 59 of the upper layer 50 of the deflection control mechanism 29 is supported by a ball bearing 48 on the rigid axle 43 fixedly held in the cage 44. The articulation shaft 47 of the lower layer 60 is held by a ball bearing 49 in a cage body 44a that is firmly connected to the cage 44. In an alternative embodiment (not shown) the articulation shafts 46, 47 may be coupled to respective electric motors, such that the sprockets 59, 69 can be motor-driven for operating the deflection control mechanism 29 in a motorized or robotic way.

As is further depicted in Fig. 5, the upper layer 50 of the deflection control mechanism comprises a metal plate 71 made of stainless steel, for example, an upper plastic plate 70, and a lower plastic plate 72 stacked upon one another. On top of the upper plastic plate 70 a top plate of the housing 45 is arranged. The plates 70, 71, 72 are fixed to each other by a vertical bolt 73 and, including the housing 45, by a screw 74. The lower layer 60 is separated from the upper layer 50 by a thin metal sheet 75 and comprises a further plastic plate 76 and a further metal plate 77, which are likewise stacked upon one another and fixed to each other by the bolt 73 and the screw 74. Further the arrangement of stacked plates is fixed to an elevated section of the handpiece body 40 by means of the bolt 73 and the screw 74. Thus, the arrangement of stacked plates of the upper and the lower layers 50, 60 forms a rigid unit, being rigidly fixed to the body 40 of the handpiece 20. Further, the cage 44 and the cage body 44a are firmly held to the body 40 by a screw 44b, and the cage 44 is also supported in a longitudinal direction by directly abutting on the housing 45, forming a rigid support structure including the cage and cage body 44, 44a, the axle 43, the stack of plates 70, 71, 72, 75, 76, 77, and the body 40.

As can be seen in Fig. 4, the metal plate 71 of the upper layer 50 exhibits a step or shoulder 80 protruding in a transverse direction, thus forming as a fixed stop limiting the movement of the stopping block 56. Similarly, a shoulder 80' on the opposing side of the metal plate 71 forms a fixed stop limiting movement of the stopping block 56'. As described above, the metal plate 71 is enclosed in a rigid package of plates stacked upon one another, the package being rigidly connected to the cage 44 supporting the sprocket 59, such that the shoulders 80, 80' and the stopping blocks 56, 56' cooperate by contact one another to form a well-defined stop limiting the movement range of the first pair of control wires 37, 37'.

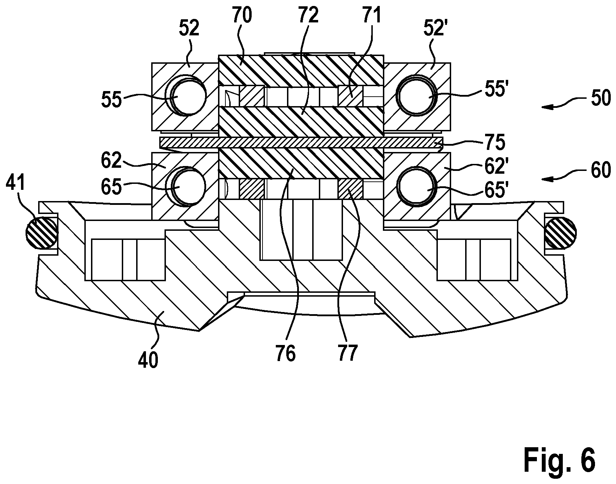

The arrangement of stacked plates is shown in a vertical cross section drawn perpendicular to the longitudinal direction, cutting through the fixation blocks 52, 52', as depicted in Fig. 6. The plates 70, 71, 72 of the upper layer 50 form an essentially rectangular block, with the fixation blocks 52, 52' being guided on the lateral sides of the plastic plates 70, 72. Thus rotation of the fixation blocks 52, 52' is avoided, while the plastic plates 70, 72 provide guidance with low friction. Likewise, the stopping blocks 56, 56' are guided by the lateral sides of the plastic plates 70, 72, thus inhibiting rotation while providing low friction. In a similar manner the fixation blocks 62, 62' and stopping blocks of the lower layer 60 are guided at the lateral sides of the plastic plate 76 of the lower layer 60. Preferably the fixation blocks 52, 52', 62, 62', as well as the stopping blocks are made of metal, for example brass, thus further reducing friction with the plastic plates 70, 72, 76. The arrangement of stacked plates 70, 71, 72 of the upper layer 50, the intermediate metal sheet 75, and the plates 76, 77 of the lower layer 60 are supported on and rigidly fixed to the body 40 of the housing 21. Fig. 6 also shows the seal ring 41 for forming a sealed enclosure when the upper part of the housing 21 has been mounted (see Fig. 1).

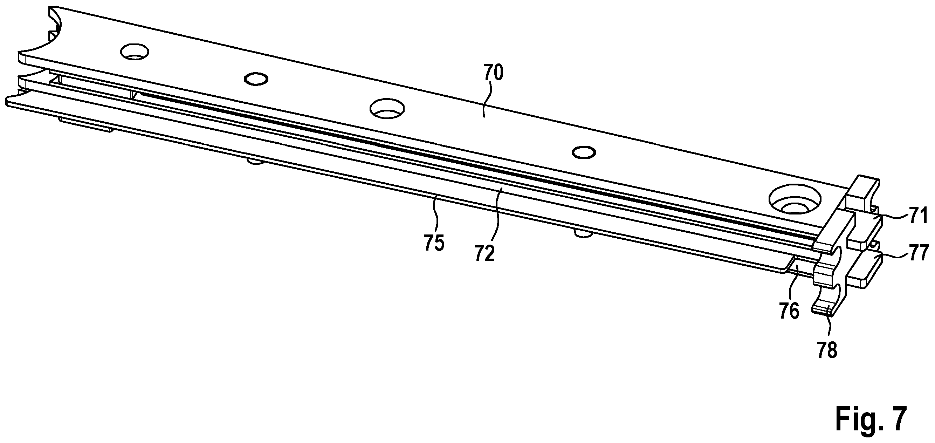

Fig. 7 shows, in an oblique view, the stacked arrangement of plates 70, 71, 72, 75, 76, 77. On a distal side, the plates are held in a frame element 78 made of stainless steel, for example. On a proximal side, the plates exhibit recesses for accommodating the sprockets 59, 69 and the shafts 46, 47.

In the above description the terms "upper" and "lower" relate to the orientation of the handpiece 20 and the deflection control mechanism 29 as shown in the figures. Any other orientation may be chosen by a user, according to requirements during use.

For clarity not all reference numerals are displayed in all figures. If a reference numeral is not explicitly mentioned in the description of a figure, it has the same meaning as in the other figures.

### Reference numerals

- 10: Flexible endoscope
- 20: Handpiece
- 21: Housing
- 22: Hand wheel
- 23: Hand wheel
- 24: Knob
- 25: Button
- 26: Connector
- 27: Light cable
- 28: Instrument port
- 29: Deflection control mechanism
- 30: Shaft
- 31: Steerable section
- 32: End cap
- 33: Pivoting element
- 35, 35': Sheath
- 36, 36': Sheath
- 37, 37': Control wire
- 38: Control wire
- 40: Body
- 41: Seal ring
- 42: Lever
- 43: Axle
- 44: Cage
- 44a: Cage body
- 44b: Screw
- 45: Half-shell housing
- 46: Shaft
- 47: Shaft
- 48: Ball bearing
- 49: Ball bearing
- 50: Upper layer
- 51, 51': Wire coil mounting
- 52, 52': Block
- 53, 53': Bore
- 54, 54': Ferrule
- 55, 55': Rod
- 56, 56': Block
- 57, 57': Hinge
- 58: Chain
- 59: Sprocket
- 60: Lower layer
- 61, 61': Wire coil mounting
- 62, 62': Block
- 65: Rod
- 66: Block
- 67: Hinge
- 68: Chain
- 69: Sprocket
- 70: Plastic plate
- 71: Metal plate
- 72: Plastic plate
- 73: Bolt
- 74: Screw
- 75: Metal Sheet
- 76: Plastic plate
- 77: Metal plate
- 78: Frame element
- 80, 80': Shoulder

## Claims

1. Deflection control mechanism for a steerable flexible endoscope (10), the endoscope (10) having an elongate flexible shaft (30) comprising a steerable section (31) that is deflectable by movement of at least one pair of counteracting control wires (37, 37', 38), the deflection control mechanism (29) comprising a support structure, a drive wheel rotatably supported in or on the support structure, and an elongate traction element having a flexible section, a first coupling section connected to a first end of the flexible section, and a second coupling section connected to a second end of the flexible section, wherein the flexible section of the traction element engages with the drive wheel, wherein the first and second coupling sections each comprise a fixation element for fixing a respective proximal end of each wire of the at least one pair of control wires (37, 37', 38), and wherein at least one of the first and second coupling sections comprises a movable stop element co-operating with a fixed stop of the support structure, wherein the movable stop element is adjustably connected to the fixation element of the respective coupling section, **characterized in that** the movable stop element is mounted on a threaded rod (55, 55', 65) connected to the fixation element of the respective coupling section.

2. Deflection control mechanism according to claim 1, **characterized in that** the movable stop element is configured as a rectangular block (56, 56', 66).

3. Deflection control mechanism according to any one of claims 1 or 2, **characterized in that** the at least one of the first and second coupling sections has a tensioning mechanism for pre-tensioning the at least one pair of control wires (37, 37', 38).

4. Deflection control mechanism according to any one of the preceding claims, **characterized in that** the support structure comprises at least one base plate arranged substantially in a base plane extending at least approximately perpendicular to a rotational axis of the drive wheel.

5. Deflection control mechanism according to claim 4, **characterized in that** the flexible section and the first and second coupling sections of the traction element extend substantially in or adjacent to the base plane and that the base plate is arranged substantially between the first and second coupling sections, the base plate supporting the fixed stop of the support structure.

6. Deflection control mechanism according to claim 4 or 5, **characterized in that** the support structure further comprises a cage structure in which the drive wheel is rotatably supported, and a housing (45) connected to the cage structure, the housing (45) being rigidly connected to the base plate.

7. Deflection control mechanism according to any one of claims 4-6, **characterized in that** the base plate is a metal plate (71, 77) and that the support structure comprises at least one plastic plate (70, 72, 76) arranged substantially parallel and adjacent to the base plate.

8. Deflection control mechanism according to claim 7, **characterized in that** the base plate and the at least one plastic plate (70, 72, 76) are rigidly connected to one another.

9. Deflection control mechanism according to claim 7 or 8, **characterized in that** the at least one plastic plate (70, 72, 76) forms a guideway for the movement of the movable stop element of the at least one of the first and second coupling sections.

10. Deflection control mechanism according to any one of the preceding claims, **characterized in that** the deflection control mechanism comprises a further drive wheel rotatably supported in the support structure, and a further elongate traction element having a flexible section, a first coupling section connected to a first end of the flexible section, and a second coupling section connected to a second end of the flexible section, wherein the flexible section of the further traction element engages with the further drive wheel, wherein the first and second coupling sections of the further traction element each comprise a fixation element for fixing a respective proximal end of each wire of at least one further pair of control wires (37, 37', 38), wherein at least one of the first and second coupling sections of the further traction element comprises a movable stop element co-operating with a further fixed stop of the support structure and being adjustably connected to the fixation element of the respective coupling section.

11. Deflection control mechanism according to claim 10 in combination with claim 5, **characterized in that** the support structure comprises a further base plate arranged approximately in a further base plane extending substantially perpendicular to a rotational axis of the further drive wheel, wherein the flexible section and the first and second coupling sections of the further traction element extend substantially in or adjacent to the further base plane, the further base plate being arranged substantially between the first and second coupling sections and supporting the further fixed stop of the support structure, and wherein the further base plate is arranged substantially parallel to and rigidly connected to the base plate.

12. Steerable flexible endoscope having an elongate flexible shaft (30) comprising a steerable section (31) that is deflectable by movement of at least one pair of counteracting control wires (37, 37', 38), further comprising a deflection control mechanism (29) in accordance with any one of the preceding claims.

13. Endoscope assembly set, comprising at least a first and a second elongate flexible shafts (30), each elongate flexible shaft (30) comprising a steerable section (31) that is deflectable by movement of at least one pair of counteracting control wires (37, 37', 38), the first and second elongate shafts (30) having different permissible ranges of longitudinal movement of the control wires (37, 37', 38), the endoscope assembly set further comprising a deflection control mechanism (29) adaptable for controlling deflection of the steerable section (31) of any one of the elongate flexible shafts (30), the deflection control mechanism (29) being configured in accordance with any one of claims 1-11.

14. Method for assembling a flexible endoscope (10), the flexible endoscope (10) having an elongate flexible shaft (30) comprising a steerable section (31) that is deflectable by movement of at least one pair of counteracting control wires (37, 37', 38), wherein the elongate flexible shaft (30) is provided, a deflection control mechanism (29) is provided, the deflection control mechanism (29) being configured in accordance with any one of claims 1-11, wherein a longitudinal position of the movable stop element is adjusted such that the deflection control mechanism (29) is adapted for controlling deflection of the steerable section (31) of the elongate flexible shaft (30), and the deflection control mechanism (29) is connected to a proximal end of the elongate flexible shaft (30).

## Patentansprüche

1. Ablenkungssteuerungsmechanismus für ein steuerbares flexibles Endoskop (10), wobei das Endoskop (10) einen länglichen, flexiblen Schaft (30) aufweist, der einen lenkbaren Abschnitt (31) umfasst, der durch die Bewegung von mindestens einem Paar gegenwirkender Steuerungsdrähte (37, 37', 38) ablenkbar ist, wobei der Ablenkungssteuerungsmechanismus (29) eine Trägerstruktur, ein Antriebsrad, das drehbar in oder an der Trägerstruktur gelagert ist, und ein längliches Zugelement mit einem flexiblen Abschnitt umfasst, wobei ein erster Koppelabschnitt mit einem ersten Ende des flexiblen Abschnitts gekoppelt ist, und ein zweiter Koppelabschnitt mit einem zweiten Ende des flexiblen Abschnitts gekoppelt ist, wobei der flexible Abschnitt des Zugelements mit dem Antriebsrad in Eingriff steht, wobei der erste und der zweite Koppelabschnitt jeweils ein Fixierelement zum Fixieren eines jeweiligen proximalen Endes jedes Drahts des mindestens einen Paars von Steuerungsdrähten (37, 37', 38) umfassen, und wobei mindestens einer des ersten und zweiten Koppelabschnitts ein bewegliches Anschlagelement umfasst, das mit einem feststehenden Anschlag der Trägerstruktur zusammenwirkt, wobei das bewegliche Anschlagelement einstellbar mit dem Fixierelement des jeweiligen Koppelabschnitts verbunden ist, **dadurch gekennzeichnet, dass** das bewegliche Anschlagelement an einer Gewindestange (55, 55', 65) montiert ist, die mit dem Fixierelement des jeweiligen Koppelabschnitts verbunden ist.

2. Ablenkungssteuerungsmechanismus nach Anspruch 1, **dadurch gekennzeichnet, dass** das bewegliche Anschlagelement als ein rechteckiger Block (56, 56', 66) konfiguriert ist.

3. Ablenkungssteuerungsmechanismus nach einem der Ansprüche 1 oder 2, **dadurch gekennzeichnet, dass** der mindestens eine des ersten und zweiten Koppelabschnitts einen Spannmechanismus zum Vorspannen des mindestens einen Paares von Steuerungsdrähten (37, 37', 38) aufweist.

4. Ablenkungssteuerungsmechanismus nach einem der vorhergehenden Ansprüche, **dadurch gekennzeichnet, dass** die Trägerstruktur mindestens eine Basisplatte umfasst, die im Wesentlichen in einer sich mindestens annähernd senkrecht zu einer Drehachse des Antriebsrades erstreckenden Basisebene angeordnet ist.

5. Ablenkungssteuerungsmechanismus nach Anspruch 4, **dadurch gekennzeichnet, dass** sich der flexible Abschnitt und der erste und der zweite Koppelabschnitt des Zugelements im Wesentlichen in oder benachbart zu der Basisebene erstrecken und dass die Basisplatte im Wesentlichen zwischen dem ersten und dem zweiten Koppelabschnitt angeordnet ist, wobei die Basisplatte den fixierten Anschlag der Trägerstruktur trägt.

6. Ablenkungssteuerungsmechanismus nach Anspruch 4 oder 5, **dadurch gekennzeichnet, dass** die Trägerstruktur ferner eine Käfigstruktur, in der das Antriebsrad drehbar getragen wird, und ein Gehäuse (45) umfasst, das mit der Käfigstruktur verbunden ist, wobei das Gehäuse (45) starr mit der Basisplatte verbunden ist.

7. Ablenkungssteuerungsmechanismus nach einem der Ansprüche 4-6, **dadurch gekennzeichnet, dass** die Basisplatte eine Metallplatte (71, 77) ist und dass die Trägerstruktur mindestens eine Kunststoffplatte (70, 72, 76) umfasst, die im Wesentlichen parallel und benachbart zu der Basisplatte angeordnet ist.

8. Ablenkungssteuerungsmechanismus nach Anspruch 7, **dadurch gekennzeichnet, dass** die Basisplatte und die mindestens eine Kunststoffplatte (70, 72, 76) starr miteinander verbunden sind.

9. Ablenkungssteuerungsmechanismus nach Anspruch 7 oder 8, **dadurch gekennzeichnet, dass** die mindestens eine Kunststoffplatte (70, 72, 76) eine Führungsbahn für die Bewegung des beweglichen Anschlagelements des mindestens einen des ersten und zweiten Koppelabschnitts bildet.

10. Ablenkungssteuerungsmechanismus nach einem der vorhergehenden Ansprüche, **dadurch gekennzeichnet, dass** der Ablenkungssteuerungsmechanismus ein weiteres Antriebsrad, das drehbar in der Trägerstruktur gelagert ist, und ein weiteres längliches Zugelement mit einem flexiblen Abschnitt umfasst, wobei ein erster Koppelabschnitt mit einem ersten Ende des flexiblen Abschnitts gekoppelt ist, und ein zweiter Koppelabschnitt mit einem zweiten Ende des flexiblen Abschnitts gekoppelt ist, wobei der flexible Abschnitt des weiteren Zugelements mit dem weiteren Antriebsrad in Eingriff steht, wobei der erste und der zweite Koppelabschnitt des weiteren Zugelements jeweils ein Fixierelement zum Fixieren eines jeweiligen proximalen Endes jedes Drahts von mindestens einem weiteren Paar von Steuerungsdrähten (37, 37', 38) umfassen, wobei mindestens einer des ersten und zweiten Koppelabschnitts des weiteren Zugelements ein bewegliches Anschlagelement umfasst, das mit einem weiteren feststehenden Anschlag der Trägerstruktur zusammenwirkt und einstellbar mit dem Fixierelement des jeweiligen Koppelabschnitts verbunden ist.

11. Ablenkungssteuerungsmechanismus nach Anspruch 10 in Verbindung mit Anspruch 5, **dadurch gekennzeichnet, dass** die Trägerstruktur eine weitere Basisplatte umfasst, die annähernd in einer weiteren sich im Wesentlichen senkrecht zu einer Drehachse des weiteren Antriebsrades erstreckenden Basisebene angeordnet ist, wobei sich der flexible Abschnitt und der erste und zweite Koppelabschnitt des weiteren Zugelements im Wesentlichen in oder benachbart zu der weiteren Basisebene erstrecken, wobei die weitere Basisplatte im Wesentlichen zwischen dem ersten und dem zweiten Koppelabschnitt angeordnet ist und den weiteren feststehenden Anschlag des Trägers trägt, und wobei die weitere Basisplatte im Wesentlichen parallel zu der Basisplatte angeordnet und starr mit dieser verbunden ist.

12. Lenkbares, flexibles Endoskop mit einem länglichen, flexiblen Schaft (30), der einen lenkbaren Abschnitt (31) umfasst, der durch die Bewegung von mindestens einem Paar gegenwirkender Steuerungsdrähte (37, 37', 38) ablenkbar ist, ferner umfassend einen Ablenkungssteuerungsmechanismus (29) nach einem der vorhergehenden Ansprüche.

13. Endoskopanordnungssatz, umfassend mindestens einen ersten und einen zweiten länglichen flexiblen Schaft (30), wobei jeder länglicher, flexibler Schaft (30) einen lenkbaren Abschnitt (31) umfasst, der durch die Bewegung von mindestens einem Paar gegenwirkender Steuerungsdrähte (37, 37', 38) ablenkbar ist, wobei der erste und der zweite längliche Schaft (30) unterschiedliche zulässige Bereiche für die Längsbewegung der Steuerungsdrähte (37, 37', 38) aufweisen, wobei der Endoskopanordnungssatz ferner einen Ablenkungssteuermechanismus (29) umfasst, der zum Steuern der Ablenkung des lenkbaren Abschnitts (31) eines der länglichen flexiblen Schäfte (30) geeignet ist, wobei der Ablenkungssteuermechanismus (29) nach einem der Ansprüche 1-11 konfiguriert ist.

14. Verfahren zur Montage eines flexiblen Endoskops (10), wobei das flexible Endoskop (10) einen länglichen, flexiblen Schaft (30) aufweist, der einen lenkbaren Abschnitt (31) umfasst, der durch die Bewegung von mindestens einem Paar gegenwirkender Steuerungsdrähte (37, 37', 38) ablenkbar ist, wobei der längliche flexible Schaft (30) bereitgestellt wird, ein Ablenkungssteuermechanismus (29) bereitgestellt wird, wobei der Ablenkungssteuermechanismus (29) nach einem der Ansprüche 1-11 konfiguriert ist, wobei eine Längsposition des beweglichen Anschlagelements derart eingestellt ist, dass der Ablenkungssteuerungsmechanismus (29) zum Steuern der Ablenkung des lenkbaren Abschnitts (31) des länglichen flexiblen Schafts (30) angepasst ist, und der Ablenkungssteuerungsmechanismus (29) mit einem proximalen Ende des länglichen flexiblen Schafts (30) verbunden ist.

## Revendications

1. Mécanisme de commande de déviation pour un endoscope flexible orientable (10), l'endoscope (10) ayant un arbre flexible allongé (30) comprenant une section orientable (31) qui peut être déviée par un mouvement d'au moins une paire de fils de commande antagonistes (37, 37', 38), le mécanisme de commande de déviation (29) comprenant une structure de support, une roue d'entraînement supportée de manière rotative dans ou sur la structure de support, et un élément de traction allongé ayant une section flexible, une première section de couplage reliée à une première extrémité de la section flexible, et une seconde section de couplage reliée à une seconde extrémité de la section flexible, dans lequel la section flexible de l'élément de traction vient en prise avec la roue d'entraînement, dans lequel les première et seconde sections de couplage comprennent chacune un élément de fixation pour fixer une extrémité proximale respective de chaque fil de l'au moins une paire de fils de commande (37, 37', 38), et dans lequel au moins l'une des premières et secondes sections de couplage comprend un élément de butée mobile coopérant avec une butée fixe de la structure de support, dans lequel l'élément de butée mobile est relié de manière ajustable à l'élément de fixation de la section de couplage respective, **caractérisé en ce que** l'élément de butée mobile est monté sur une tige filetée (55, 55', 65) reliée à l'élément de fixation de la section de couplage respective.

2. Mécanisme de commande de déviation selon la revendication 1, **caractérisé en ce que** l'élément de butée mobile est conçu comme un bloc rectangulaire (56, 56', 66).

3. Mécanisme de commande de déviation selon l'une quelconque des revendications 1 ou 2, **caractérisé en ce que** l'au moins une des première et seconde sections de couplage a un mécanisme de tension pour pré-tendre l'au moins une paire de fils de commande (37, 37', 38).

4. Mécanisme de commande de déviation selon l'une quelconque des revendications précédentes, **caractérisé en ce que** la structure de support comprend au moins une plaque de base agencée sensiblement dans un plan de base s'étendant au moins approximativement perpendiculairement à un axe de rotation de la roue d'entraînement.

5. Mécanisme de commande de déviation selon la revendication 4, **caractérisé en ce que** la section flexible et les première et seconde sections de couplage de l'élément de traction s'étendent sensiblement dans le ou adjacentes au plan de base et que la plaque de base est agencée sensiblement entre les première et seconde sections de couplage, la plaque de base supportant la butée fixe de la structure de support.

6. Mécanisme de commande de déviation selon la revendication 4 ou 5, **caractérisé en ce que** la structure de support comprend en outre une structure de cage dans laquelle la roue d'entraînement est supportée de manière rotative, et un boîtier (45) relié à la structure de cage, le boîtier (45) étant relié de manière rigide à la plaque de base.

7. Mécanisme de commande de déviation selon l'une quelconque des revendications 4 à 6, **caractérisé en ce que** la plaque de base est une plaque métallique (71, 77) et que la structure de support comprend au moins une plaque en plastique (70, 72, 76) agencée de manière sensiblement parallèle et adjacente à la plaque de base.

8. Mécanisme de commande de déviation selon la revendication 7, **caractérisé en ce que** la plaque de base et l'au moins une plaque en plastique (70, 72, 76) sont reliées de manière rigide l'une à l'autre.

9. Mécanisme de commande de déviation selon la revendication 7 ou 8, **caractérisé en ce que** l'au moins une plaque en plastique (70, 72, 76) forme une voie de guidage pour le mouvement de l'élément de butée mobile de l'au moins une des première et seconde sections de couplage.

10. Mécanisme de commande de déviation selon l'une quelconque des revendications précédentes, **caractérisé en ce que** le mécanisme de commande de déviation comprend une autre roue d'entraînement supportée de manière rotative dans la structure de support, et un autre élément de traction allongé ayant une section flexible, une première section de couplage reliée à une première extrémité de la section flexible, et une seconde section de couplage reliée à une seconde extrémité de la section flexible, dans lequel la section flexible de l'autre élément de traction vient en prise avec l'autre roue d'entraînement, dans lequel les première et seconde sections de couplage de l'autre élément de traction comprennent chacune un élément de fixation pour fixer une extrémité proximale respective de chaque fil d'au moins une autre paire de fils de commande (37, 37', 38), dans lequel au moins l'une des première et seconde sections de couplage de l'autre élément de traction comprend un élément de butée mobile coopérant avec une autre butée fixe de la structure de support et étant relié de manière ajustable à l'élément de fixation de la section de couplage respective.

11. Mécanisme de commande de déviation selon la revendication 10 en combinaison avec la revendication 5, **caractérisé en ce que** la structure de support comprend une autre plaque de base agencée approximativement dans un autre plan de base s'étendant sensiblement perpendiculairement à un axe de rotation de l'autre roue d'entraînement, dans lequel la section flexible et les première et seconde sections de couplage de l'autre élément de traction s'étendent sensiblement dans ou adjacentes à l'autre plan de base, l'autre plaque de base étant agencée sensiblement entre les première et seconde sections de couplage et supportant l'autre butée fixe de la structure de support, et dans lequel l'autre plaque de base est agencée sensiblement parallèlement à et rigidement reliée à la plaque de base.

12. Endoscope flexible orientable ayant un arbre flexible allongé (30) comprenant une section orientable (31) qui peut être déviée par un mouvement d'au moins une paire de fils de commande antagonistes (37, 37', 38), comprenant en outre un mécanisme de commande de déviation (29) conformément à l'une quelconque des revendications précédentes.

13. Ensemble d'assemblage d'endoscope, comprenant au moins un premier et un second arbre flexible allongé (30), chaque arbre flexible allongé (30) comprenant une section orientable (31) qui peut être déviée par un mouvement d'au moins une paire de fils de commande antagonistes (37, 37', 38), les premier et second arbres allongés (30) ayant des plages admissibles différentes de mouvement longitudinal des fils de commande (37, 37', 38), l'ensemble d'assemblage d'endoscope comprenant en outre un mécanisme de commande déviation (29) pouvant être adapté pour commander une déviation de la section orientable (31) de l'un quelconque des arbres flexibles allongés (30), le mécanisme de commande de déviation (29) étant conçu conformément à l'une quelconque des revendications 1 à 11.

14. Procédé d'assemblage d'un endoscope flexible (10), l'endoscope flexible (10) ayant un arbre flexible allongé (30) comprenant une section orientable (31) qui peut être déviée par un mouvement d'au moins une paire de fils de commande antagonistes (37, 37', 38), dans lequel l'arbre flexible allongé (30) est fourni, un mécanisme de commande de déviation (29) est fourni, le mécanisme de commande de déviation (29) est conçu conformément à l'une quelconque des revendications 1 à 11, dans lequel une position longitudinale de l'élément de butée mobile est ajustée de telle sorte que le mécanisme de commande de déviation (29) est adapté pour commander une déviation de la section orientable (31) de l'arbre flexible allongé (30), et le mécanisme de commande de déviation (29) est relié à une extrémité proximale de l'arbre flexible allongé (30).
